# EUROPEAN PATENT SPECIFICATION

(11) **EP 0 965 267 B1**
(45) Date of publication and mention of the grant of the patent: **10.07.2002**
(21) Application number: 99105951.0
(22) Date of filing: 24.03.1999
(51) Int. Cl.: A01M 1/20, A01M 13/00, A61L 9/03, H05B 3/14

(54) **PTC heating device for insecticides or perfumes**
PTC Verdampfer für Insektizide und Duftstoffe
Vaporisateur du type PCT pour des insecticides et des parfumes

(30) Priority: 26.03.1998 IT MI980217
(43) Date of publication of application: 22.12.1999
(73) Proprietor: ZOBELE INDUSTRIE CHIMICHE S.p.A., I-38100 Trento (IT)
(72) Inventor: Zobele, Franco, 38100 Trento (IT); Ambrosi, Stefano, 38014 Gardolo (IT)
(74) Representative: Faggioni, Marco, Dr. Ing.

(56) References cited:
- EP-A- 0 290 159
- WO-A-97/02054

## Description

The present invention relates to a heating device intended for vaporizers used with insecticides or perfumes, of the type where the heating element is a posistor, i.e. a PTC (Positive Temperature Coefficient) heating element suitable both for exclusive heating of either solids or liquids formulation or both (so-called dual-purpose vaporizer). More particularly the invention relates to the system for fixing the PTC element inside the heating device and also to a particular design of this device suitable for ensuring more uniform heating.

As it is well-known to persons skilled in the art, PTC elements have gradually taken the place of conventional metal-wire electric resistors, despite their higher cost, since they are decidedly advantageous compared to the latter. In fact, PTC elements consist of tablets of pressed conductive granular material having the electrical property of a decreasing resistivity with an increase in temperature, unlike that which happens instead in conventional metal-wire resistors. As a result of this characteristic, the heat produced by the Joule effect in PTC elements never increases beyond a predetermined level and this therefore makes them particularly suitable for simple applications - i.e. applications in which there are no other temperature control systems - where the heat produced must remain as constant as possible over time and where it is necessary to ensure that the device is protected as far as possible against overheating.

The electrical connection to the PTC elements must involve the whole area of the two opposite surfaces of the tablet and for this reason these surfaces are almost always entirely metallized, such that the electrical contact with the power supply leads may occur at any point of said surfaces. This electrical contact may be performed by means of welding or by means of contact with metal plates adhering to the surfaces of the PTC element, which are in turn connected to the electrical power supply leads by means of welding or riveting. The use of metal plates is, however, widely preferred because it allows faster and cheaper assembly and also because it is less prone to malfunctions than in the case of welding joints. The present invention is specifically intended to improve an electrical connection system for the PTC element of the preferred type described above, i.e. a system which uses metal contact plates. The invention also relates to a particular design of the heating device, as a result of which it is possible to obtain a homogeneous distribution of heat when the heating device is used with solid substances in the form of mats.

The main problem which arises with a system for the electrical connection of the PTC element using metal plates is that said plates must be able to remain in close contact with the PTC element despite knocks, vibrations, thermal expansion and the like, to which the vaporizer may be exposed during its working life. For this reason, for some time now, it has become widespread to use metal contact plates of the type in which at least one plate is provided with an elastic contact against the corresponding surface of the PTC element, and this elastic contact is kept permanently against the surface of the PTC element by pressing the plate against the PTC element and fixing it in the desired compressed position using suitable closing means, i.e. a cover, for the seat in which the PTC element is housed. Said cover must therefore be able to be securely fixed onto its seat - using mechanical means or by means of adhesives - so as to ensure that the elastic reaction force exerted on it by the plate does not push it away from the latter, thus resulting in an unstable electrical contact of the plate against the PTC element. An example of this kind of device to fix in position the PTC element, and the electric contact metal plates in their seat, is disclosed in EP-A-290159.

The present Applicants have introduced an improvement with regard to this state of the art, as disclosed in WO 97/02054, the contents of which are intended as being incorporated here by way of reference, whereby the plate arranged towards the opening of the receiving seat for the PTC element, namely between said PTC element and the cover of its seat, has elastic engaging means, in the form of thin folded-back tongues which project from the periphery of the plate and are formed integrally therewith and which are intended to engage into the seat for the PTC element when the cover is fitted in position. According to the teachings of this publication, the PTC element is therefore kept in position as a result of the elastic force exerted by the metal plate arranged between the PTC element and the cover against the walls of the seat of said PTC element, when the plate itself has been enclosed in said seat following the closing of the cover. The presence of the cover is necessary, in the embodiment shown in said patent, to ensure that the plate is kept uniformly pressed inside the seat and that its tongues simultaneously grip on the edges of said seat, i.e. in the boundary zone between cover and seat. Said cover therefore no longer performs, as in the case of the covers of known vaporizers, the function of a mechanical retainer against the elastic force of the plate - this force being discharged onto the external edges of the seat of the PTC - but is used as an assembly tool for ensuring the simultaneous gripping of all the tongues and remains in its seat as a safety means for preventing one of said tongues from becoming disengaged from the edge of the seat of the PTC element as a result of vibrations, knocks and the like, thus making the assembly unstable.

From subsequent improvement research on the subject of said patent - still with the aim of simplifying the structure of the heater and speeding up and automating assembly thereof - the Applicants have finally discovered that by forming the seat of the PTC element so that it has a depth which is markedly greater than the thickness of the package consisting of the two contact plates and the PTC element arranged therebetween - instead of a depth which is substantially equal to said thickness, as was the case in the abovementioned patent and in the known prior art - the presence of the cover is no longer needed in any way either during assembly or during use of the vaporizer, thus resulting in a further important simplification of the structure of the vaporizer and assembly thereof. With this new constructional design in fact, the elastic tongues of the plate arranged towards the opening of the seat no longer engage with the external edge of the seat, but with the internal walls thereof; assembly is therefore able to occur also in not perfectly level conditions, whereas any disengagement of the contact plate from the seat is prevented by the fact that each of the tongues has a stable point of equilibrium against the wall of the seat for the PTC element.

The present invention therefore relates to a heating device for vaporizers used with insecticides or perfumes, of the type comprising:
a support body made of heat-resistant material and having a seat for a heating element, a flat surface for supporting a mat of a solid formulation and, in case, a hole or a saddle seat for housing a wick of a liquid formulation;
and at least one PTC heating element, housed in said seat, contacted on its opposite faces by metal conducting plates connected to the terminals of an electric power supply circuit;
characterized in that the plate arranged at the opening of said seat has peripheral elastic means apt to engage with the internal side walls of said seat and in that the depth of said seat is greater than the thickness of the package formed by the PTC element and the two metal plates contacting the same.

According to another feature of the present invention, in order to obtain a better distribution of the heat, said flat surface for supporting a mat of a solid formulation has an ellipsoidal hollow, the centre of which is located above the centre of said PTC element.

Further features and advantages of the present invention will emerge, anyway, more clearly from the following detailed description of preferred embodiments thereof, in which:
Fig. 1 is a plan view of a vaporizer according to the present invention, in the embodiment for use with solid formulations as mats;
Fig. 2 is a cross-sectional view, along the line A-A of Fig. 1;
Fig. 3 is a plan view which shows the assembly of the vaporizer according to Fig. 1, with horizontal electrical leads;
Fig. 4 is a plan and cross-sectional view, similar to the view of Fig. 3, which shows the assembly of a second embodiment of the vaporizer, with vertical electrical leads;
Fig. 5 in a view, similar to the preceding ones, which shows the assembly of a third embodiment of the vaporizer, of the dual-purpose type, with horizontal electric leads;
Fig. 6 is a plan view which shows an embodiment of the heating device suitable for allowing a more homogeneous distribution of heat on the surface for supporting the solid formulation; and
Fig. 7 is a cross-sectional view along the line A-A of Fig. 6.

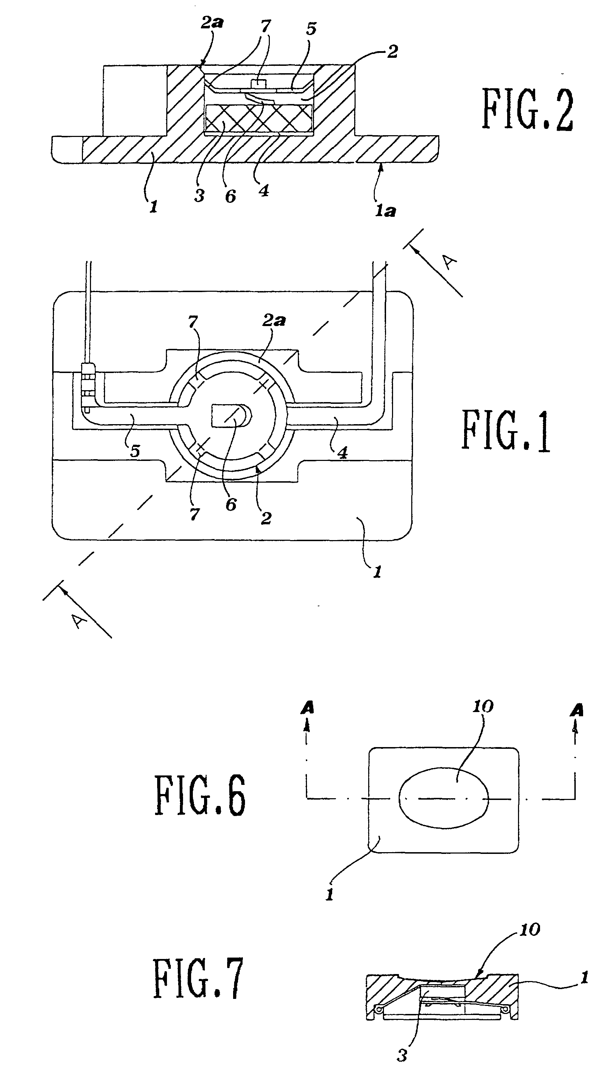

Figs. 1 and 2 clearly show the heating device according to the present invention. It comprises a support body 1 which is made of heat-resistant material, for example ceramic material or another suitable plastic material (for example based on phenylene polysulphide - FPS), having formed therein a well seat 2 for housing a PTC heating element. The well seat 2 has a cross-section suitable for housing, without a significant degree of slack, the PTC element 3. In the embodiments shown, in which the PTC element is in the form of a disk-shaped tablet, the seat 2 is cylindrical and preferably has a receiving surface 2a flared towards the outside of the support.

The electrical connection of the PTC element 3 is performed by means of two contact plates 4 and 5 and, in particular, an upper plate 4 and a lower plate 5 - the terms upper and lower referring to the normal position of use of the vaporizer in which the continuous surface 1a of the body 1 is directed upwards - which are made of metallic material having a modulus of elasticity which is sufficiently high for the purposes which will be stated below. Both the plates 4 and 5 have a circular shape, but whereas the plate 4 is flat and is arranged without slack between the bottom wall of the well seat 2 and a first face of the PTC element, the plate 5 is provided with central elastic means for contacting the second face of the PTC element and with peripheral elastic means for securing said plate 5 to the side walls of the well seat 2. These central and peripheral elastic means are preferably formed by cutting out, in the material from which the plate 5 is made, a central tongue 6 and a plurality of peripheral tongues 7. The tongues 6 and 7 are then folded-back away from the surface of the plate, respectively towards the PTC element 3 and towards the opening of the well seat 2; the dimensions of the tongues 7 must be such that, after folding back, the external diameter of the plate 5 in the region of the tongues 7 is slightly greater than the diameter of the hole of the seat 2. By way of an alternative to the arrangement illustrated above, the elastic means 6 may be formed also or exclusively on the upper contact plate 4.

With the constructional design described above, it is possible to reach easily the objects of the model, namely permanent blocking of the PTC element inside the seat 2 and a perfect and continuous electrical connection thereof. In order to achieve these objects, it is sufficient in fact to insert into the well seat 2 first of all the upper plate 4, then the PTC element 3 and finally the plate 5. Since the external diameter of the contact plate 5 in the region of the tongues 7 is greater than the diameter of the seat 2, the plate 5 must be forced into said seat, thus causing elastic flexing of the tongues 7 against the internal walls of the seat 2. This operation is facilitated by the presence of the flared receiving surface 2a, against which the tongues 7 of the plate 5 bear during the initial step of insertion. Said plate is then pushed inside the seat 2 until the central tongue 6 comes into elastic contact with the PTC element 3. In this position, the tongues 7, which are now elastically deformed against the walls of the seat 2, ensure perfect permanent blocking of the plate 5 inside the well seat 2, just thanks to the elastic force which they exert against the side walls of this seat. Furthermore, owing to their inclination, every force which tends to displace the plate 5 from its working position would increase the pressure of the tongues 7 against the walls of the seat 2, preventing any possible displacement of the plate 5. The central tongue 6 ensures, on other hand, with its elasticity, that even in the case of vibrations or knocks a continuous electrical contact is maintained between the plate 5 and the PTC element 3.

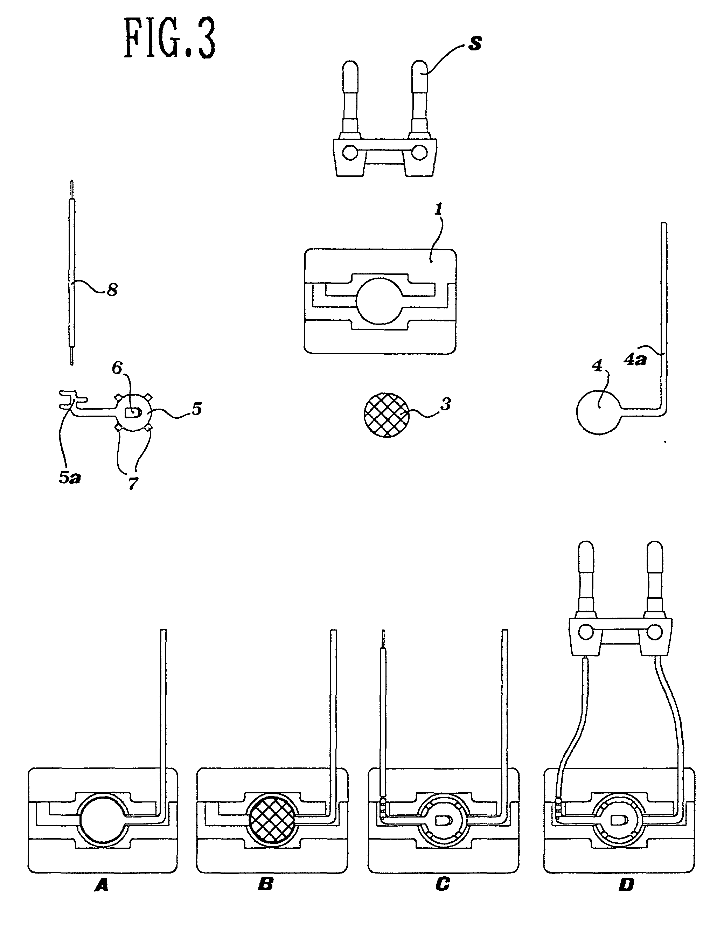

Fig. 3 shows in detail the various parts which form the heating device of the present invention and the various assembly steps (A-D), in the case of a vaporizer of the type shown in Figs. 1 and 2, i.e. provided with horizontal electrical leads.

With regard to the parts already described above, it can be seen that the plate 4 has a rod-shaped extension 4a for direct connection to the plug S, while the plate 5 is provided with end lugs 5a which are intended to be folded and riveted onto the exposed end of an electrical cable 8. Assembly of the device involves four successive steps, i.e.:
A - insertion of the upper plate 4 into the well seat 2;
B - insertion of the PTC element 3 on top of the plate 4;
C - insertion and force-fitting of the lower plate 5, together with the electrical cable 8 connected to it beforehand, inside the seat 2 and on top of the PTC element 3;
D - insertion of the ends of the cable 8 and of the rod-shaped extension 4a into the plug S.

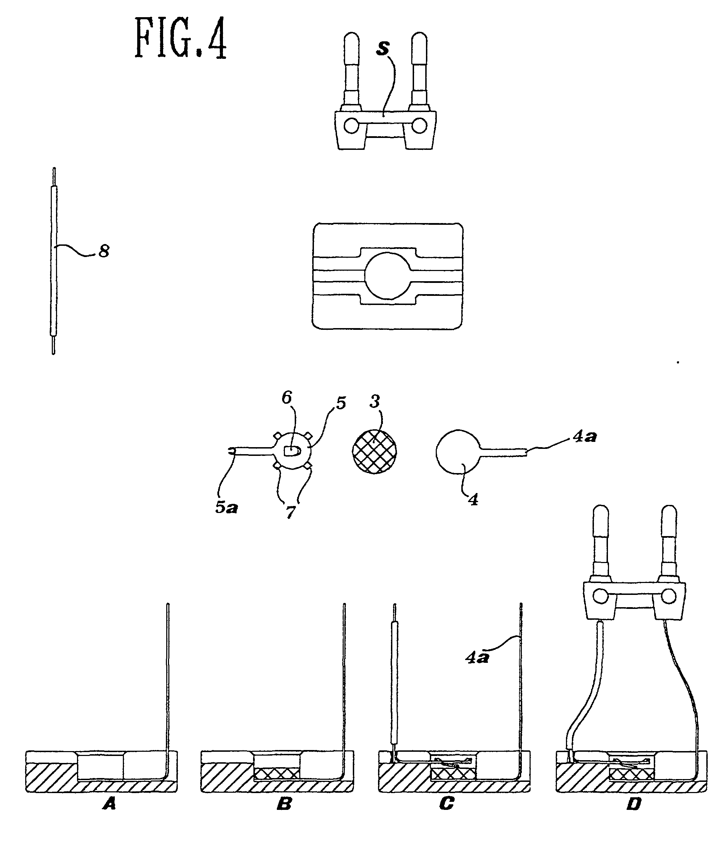

Fig. 4 shows, in a manner similar to that of Fig. 3, the parts and the assembly of a heating device according to the present invention, in the case of a vaporizer with vertical electrical leads. The differences in shape of the leads 4a and 5a of the plates 4 and 5, which are both directed upwards, as well as the different shape of the support body 1 should be noted. Assembly of the device is, however, entirely identical to that described in relation to the preceding Fig. 3.

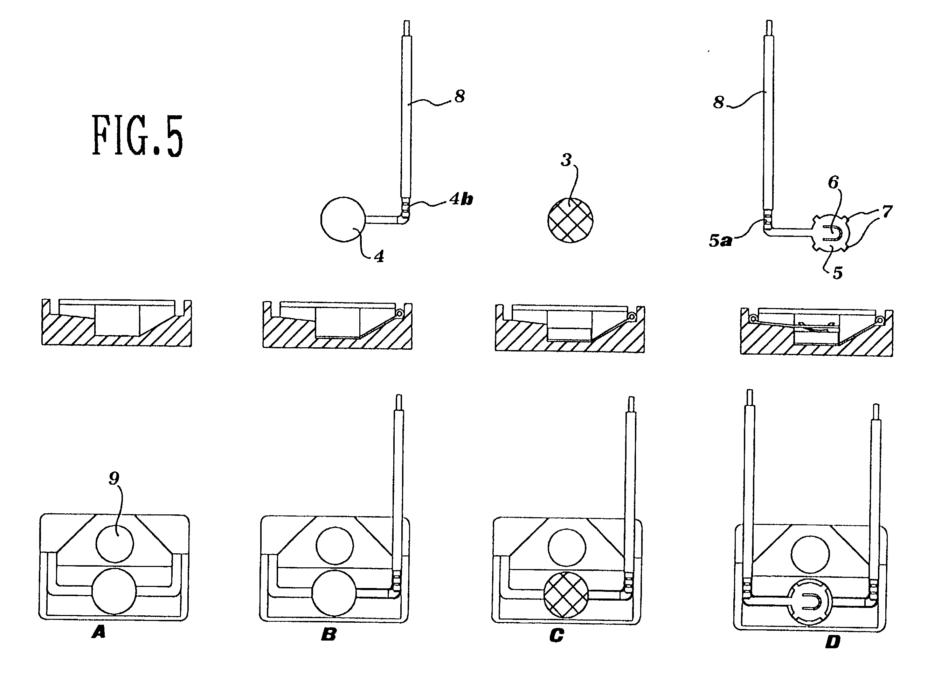

Finally, Fig. 5 shows, in a manner similar to the preceding Figs. 3 and 4, the parts and the assembly of a heating device according to the present invention, in the case of a dual-purpose vaporizer, wherein the support body 1 is provided with a hole 9 for housing the wick of a liquid formulation. In this embodiment, both the plates 4 and 5 have their ends provided with lugs 4b, 5a to which strands 8 of an electrical cable are fixed. In this case, also, assembly of the device envisages the same four steps A to D already described with reference to Fig. 3.

Fig. 6 shows a particular embodiment of the heating device or, more precisely, of the support body 1, for the purpose of obtaining better distribution of the heat when the heating device is used for solid formulation as mats. As can be clearly seen in the drawing, the support body 1 has, in a central zone of the flat surface for supporting the mat, an ellipsoidal hollow 10, the centre of which is located above the centre of the underlying PTC element 3, as can be clearly seen in the cross-sectional drawing of Fig. 7. In this way, the transmission of the heat into the zone located immediately above the PTC element is slowed down by the presence of the air chamber formed in the ellipsoidal hollow, while it is promoted in the zones which are further away from the PTC element, owing to the fact that in those zones the support body 1 - which, as already stated, has an excellent heat transmission coefficient - is directly in contact with the solid formulation as a mat.

From the above description it can be clearly understood how the present invention has fully reached the predefined object of obtaining a permanent and reliable blocking of the PTC heating element in the respective support body, without the aid of any of external fixing part, such as a cover or the like. The invention has also fully achieved its other object of obtaining a more uniform distribution of the heat for the solid formulation as mats, by means of a simple modification to the shape of the support body of the heating device. It is clear, moreover, from the various embodiments shown in the description that the objects reached by the invention are entirely independent from the shape and the type of heating device; the scope of the present invention, as defined in the accompanying claims, therefore extends to all the possible types of vaporizer with a PTC heating element, even if structurally different from those specifically illustrated in the present description.

## Claims

1. Heating device for vaporizers used with insecticides or perfumes, of the type comprising:
a support body (1) made of heat-resistant material and having a seat (2) for a heating element, a flat surface (1a) for supporting a mat of a solid formulation and, in case, a hole or a saddle seat (9) for housing a wick of a liquid formulation;
and at least one PTC heating element (3), housed in said seat (2), contacted on its opposite faces by metal conducting plates (4, 5) connected to the terminals of an electric power supply circuit;
**characterized in that** the plate (5) arranged at the opening of said seat (2) has peripheral elastic means (7) apt to engage with the interna side walls of said seat (2) and **in that** the depth of said seat (2) is greater than the thickness of the package formed by the PTC element (3) and the two metal conducting plates (4, 5) contacting the same.

2. Heating device as claimed in Claim 1, wherein at least one of said plates (4,5) has elastic means (6) apt to engage with the contact surface of said PTC element (3).

3. Heating device as claimed in Claim 1, wherein said peripheral elastic means consist of a plurality of tongues (7) which are formed integrally with the periphery of said plate (5) and are folded back towards the opening of said seat (2), the transverse dimension of the plate (5) in the region of said tongues (7) being greater than the corresponding dimension of said seat (2).

4. Heating device as claimed in Claim 2, wherein said elastic means contacting PTC element (3) consist of at least one tongue (6) which is cut out, in a central position, from said plate (5) and folded back towards the surface of the PTC element (3).

5. Device as claimed in Claim 3 and Claim 4, wherein said peripheral tongues (7) and said central tongue (6) are all formed on the contact plate (5) positioned near to the opening of said seat. (2).

6. Heating device as claimed in anyone of the preceding claims, wherein said PTC element (3) is a cylindrical tablet and said seat (2) is a cylindrical well seat.

7. Heating device as claimed in any one of the preceding claims, wherein said metal plates (4, 5) are made from a metallic material with a high modulus of elasticity.

8. Heating device as claimed in Claim 1, wherein said flat surface (1a) for supporting a mat of a solid formulation has an ellipsoidal hollow (10).

9. Heating device as claimed in Claim 8, wherein the centre of said ellipsoidal hollow (10) is located above the centre of said PTC element (3).

## Patentansprüche

1. Beheizungsvorrichtung für Verdampfer, die mit Insektiziden oder Duftstoffen verwendet werden, in der Bauart mit:
einem Tragkörper (1), der aus einem wärmebeständigen Material besteht und eine Aufnahme (2) für ein Beheizungselement aufweist, eine ebene Fläche (1a) zum Tragen einer Matte einer festen Formulierung und, ggf., mit einer Öffnung oder einer Sattelaufnahme (9) zum Aufnehmen eines Dochts einer flüssigen Formulierung;
und zumindest einem PTC-Beheizungselement (3), das in der Aufnahme (2) aufgenommen ist und auf seinen gegenüberliegenden Seitenflächen durch metallisch leitende Platten (4, 5) kontaktiert ist, die mit den Anschlüssen einer elektrischen Stromversorgungsschaltung verbunden sind;
**dadurch gekennzeichnet, daß** die Platte (5), die an der Öffnung der Aufnahme (2) angeordnet ist, auf dem Umfang angeordnete elastische Mittel (7) aufweist, die dazu bestimmt sind, mit den inneren Seitenwänden der Aufnahme (2) zusammenzuwirken, und daß die Tiefe der Aufnahme (2) größer als die Dicke des Pakets ist, das durch das PTC-Element (3) und die beiden metallisch leitenden Platten (4, 5) gebildet ist, die mit diesen in Kontakt stehen.

2. Beheizungseinrichtung nach Anspruch 1, **dadurch gekennzeichnet, daß** zumindest eine der beiden genannten Klappen (4, 5) ein elastisches Mittel (6) aufweist, das dazu bestimmt ist, mit der Kontaktfläche des PTC-Elements (3) zusammenzuwirken.

3. Beheizungseinrichtung nach Anspruch 1, **dadurch gekennzeichnet, daß** die auf dem Umfang angeordneten elastischen Mittel aus einer Anzahl von Zungen (7) bestehen, die einteilig mit dem Außenrand der genannten Platte (5) ausgebildet sind und zu der Öffnung der Aufnahme (2) zurückgebogen sind, wobei die Querabmessung der Platte (5) im Bereich der genannten Zungen (7) größer ist als die entsprechende Abmessung der Aufnahme (2).

4. Beheizungseinrichtung nach Anspruch 2, **dadurch gekennzeichnet, daß** das elastische Mittel, das das PTC-Element (3) kontaktiert, aus zumindest einer Zunge (6) besteht, die in einer zentralen Position aus der Platte (5) ausgeschnitten ist und zu der Oberfläche des PTC-Elements (3) zurückgebogen ist.

5. Beheizungseinrichtung nach Anspruch 3 und 4, **dadurch gekennzeichnet, daß** die auf dem Umfang angeordneten Zungen (7) und die zentrale Zunge (6) alle auf der Kontaktplatte (5) ausgebildet sind, die benachbart zu der Öffnung der Aufnahme (2) positioniert ist.

6. Beheizungseinrichtung nach einem der vorangehenden Ansprüche, **dadurch gekennzeichnet, daß** das PTC-Element (3) eine zylindrische Tablette ist, und daß die Aufnahme (2) eine zylindrische Schachtaufnahme ist.

7. Beheizungseinrichtung nach einem der vorangehenden Ansprüche, **dadurch gekennzeichnet, daß** die genannten Metallplatten (4, 5) aus einem metallischen Material mit einem großen Elastizitätsmodul hergestellt sind.

8. Beheizungseinrichtung nach Anspruch 1, **dadurch gekennzeichnet, daß** die ebene Fläche (1a) zum Tragen einer Matte einer festen Formulierung einen elliptischen Hohlraum (10) aufweist.

9. Beheizungseinrichtung nach Anspruch 8, **dadurch gekennzeichnet, daß** der Mittelpunkt des elliptischen Hohlraums (10) über dem Mittelpunkt des genannten PTC-Elements (3) angeordnet ist.

## Revendications

1. Dispositif de chauffage pour vaporisateurs utilisés avec des insecticides ou des parfums, du type comprenant :
un corps de support (1) constitué d'un matériau résistant à la chaleur et comportant un siège (2) pour un élément de chauffage, une surface plate (1a) pour supporter une pastille d'une formulation solide et, au cas où, un trou ou un siège façonné en forme de selle(9) pour loger une mèche d'une formulation liquide ;
et au moins un élément de chauffage CTP (3) logé dans ledit siège (2), contacté sur ses faces opposées par des plaques de métal conductrices (4, 5) reliées aux bornes d'un circuit d'alimentation électrique ;
**caractérisé en ce que** la plaque (5) située à l'ouverture dudit siège (2) comporte un moyen élastique périphérique (7) adapté à s'engager avec les parois latérales internes dudit siège (2) et **en ce que** la profondeur dudit siège (2) est supérieure à l'épaisseur de l'ensemble formé par l'élément CTP (3) et les deux plaques de métal conductrices (4, 5) contactant celui-ci.

2. Dispositif de chauffage selon la revendication 1, dans lequel au moins l'une desdites plaques (4, 5) comporte un moyen élastique (6) adapté à s'engager avec la surface de contact dudit élément CTP (3).

3. Dispositif de chauffage selon la revendication 1, dans lequel ledit moyen élastique périphérique consiste en une pluralité de languettes (7) qui sont formées d'un seul tenant avec la périphérie de ladite plaque (5) et sont repliées vers l'ouverture dudit siège (2), la dimension transversale de la plaque (5) dans la région desdites languettes (7) étant supérieure à la dimension correspondante dudit siège (2).

4. Dispositif de chauffage selon la revendication 2, dans lequel ledit moyen élastique contactant l'élément CTP (3) consiste en au moins une languette (6) qui est découpée, en une position centrale, dans ladite plaque (5) et repliée vers la surface de l'élément CTP (3).

5. Dispositif de chauffage selon la revendication 3 et la revendication 4, dans lequel lesdites languettes périphériques (7) et ladite languette centrale (6) sont toutes formées sur la plaque de contact (5) positionnée près de l'ouverture dudit siège (2).

6. Dispositif de chauffage selon l'une quelconque des revendications précédentes, dans lequel ledit élément CTP (3) est une tablette cylindrique et ledit siège (2) est un siège cylindrique en puits.

7. Dispositif de chauffage selon l'une quelconque des revendications précédentes, dans lequel lesdites plaques de métal (4, 5) sont constituées d'un matériau métallique avec un module d'élasticité élevé.

8. Dispositif de chauffage selon la revendication 1, dans lequel ladite surface plate (1a) pour supporter une pastille d'une formulation solide comporte un creux ellipsoïdal (10).

9. Dispositif de chauffage selon la revendication 8, dans lequel le centre dudit creux ellipsoïdal (10) est situé au-dessus du centre dudit élément CTP (3).
